Europäisches Patentamt

(19) European Patent Office

Office européen des brevets

(11) Publication number: **0 292 255**
**A2**

(12) **EUROPEAN PATENT APPLICATION**

(21) Application number: 88304490.1

(22) Date of filing: 18.05.88

(51) Int. Cl.⁴: **C 07 K 7/00**
**A 61 K 37/64**

(30) Priority: 22.05.87 US 53179    22.05.87 US 53181
22.05.87 US 53180

(43) Date of publication of application:
23.11.88 Bulletin 88/47

(84) Designated Contracting States:
CH DE FR GB IT LI NL

(71) Applicant: MERCK & CO. INC.
126, East Lincoln Avenue P.O. Box 2000
Rahway New Jersey 07065-0900 (US)

(72) Inventor: Freidinger, Roger
2185 Rebecca Drive
Hatfield, PA 19440 (US)

Hannah, John
155 Idlebrook Lane
Matawan, NJ 07747 (US)

Colonno, Richard
17 Brookdale Drive
New Britain, PA 18901 (US)

Stein, Robert B.
1021 Delene Road
Rydal, PA 19046 (US)

Garsky, Victor M.
752 Palmer Place
Blue Bell, PA 19433 (US)

Tolman, Richard
20 Upper Warren Way
Warren, NJ 07060 (US)

(74) Representative: Hesketh, Alan, Dr.
European Patent Department Merck & Co., Inc. Terlings
Park Eastwick Road
Harlow Essex, CM20 2QR (GB)

(54) Inhibitor of ribonucleotide reductase.

㉗ The pentapeptide Val Val Asn Asp Leu has been found to be the shortest peptide sequence that inhibits the activity of the ribonucleotide reductase enzyme of herpes simplex virus in vitro thereby inhibiting viral replication. Longer peptides also have been found that are effective inhibitors.

EP 0 292 255 A2

**Description**

## INHIBITOR OF RIBONUCLEOTIDE REDUCTASE

### ABSTRACT OF THE INVENTION

The pentapeptide Val Val Asn Asp Leu has been found to be the shortest peptide sequence that inhibits the activity of the ribonucleotide reductase enzyme of herpes simplex virus in vitro thereby inhibiting viral replication. Longer peptides also have been found that are effective inhibitors.

### BACKGROUND OF THE INVENTION

The ribonucleotide reductase enzyme of herpes simplex virus consists of 2 components which must remain associated for the enzyme to convert ribonucleotide diphosphates to deoxy ribonucleotide diphosphates. The enzyme is known to be required for herpes simplex virus replication. Dutia et al., Nature 321:439-441 (1986) and Cohen et al., Nature 321:441-443 (1986) both disclosed that the nonapeptide, Tyr Ala Gly Ala Val Val Asn Asp Leu, inhibited in vitro the activity of this enzyme. In addition Dutia et al., op. cit., also disclosed that its 8-desalanine homolog, Tyr Gly Ala Val Val Asn Asp Leu, also inhibited in vitro the activity of this enzyme.

### OBJECTS OF THE INVENTION

It is an object of the present invention to provide novel peptides which inhibit the activity of the ribonucleotide reductase enzyme of herpes simplex virus. Another object is to provide inhibitory peptides that are shorter than known peptides that inhibit this conversion. A further object is to provide novel longer peptides that inhibit the ribonucleotide reductase enzyme of herpes simplex virus. Still another object is to provide inhibitory peptides that contain a D-amino acid. These and other objects of the present invention will be apparent from the following description.

### SUMMARY OF THE INVENTION

The pentapeptide Val Val Asn Asp Leu has been found to be the shortest peptide sequence that inhibits the activity of the ribonucleotide reductase enzyme of herpes simplex virus in vitro. Longer peptides and peptides containing a D-amino acid also have been found that are effective inhibitors.

### DETAILED DESCRIPTION

It has now been found that three series of peptides are effective to inhibit the activity of the ribonucleotide reductase enzyme of herpes simplex virus in vitro. This enzyme is required for replication of the herpes simplex virus

In the present invention the amino acids listed below are identified both by conventional 3 letter and single letter abbreviations as indicated below:

| | | |
|---|---|---|
| Alanine | Ala | A |
| 5-Aminopentanoic acid | Apa | B |
| Aminoisobutyric acid | Aib | J |
| Arginine | Arg | R |
| Asparagine | Asn | N |
| Aspartic Acid | Asp | D |
| Cyclohexylphenylalanine | ChA | U |
| Cysteine | Cys | C |
| Glutamic Acid | Glu | E |
| Glycine | Gly | G |
| Histidine | His | H |
| Leucine | Leu | L |
| Lysine | Lys | K |
| Methionine | Met | M |
| Phenylalanine | Phe | F |
| Proline | Pro | P |
| Serine | Ser | S |
| Thronine | Thr | T |
| Tryptophane | Trp | W |
| Tyrosine | Tyr | Y |
| Valine | Val | V |

Other abbreviations used in the present patent application are the following:
Ac  acetyl
MeO  O-methyl
Bzl  benzyl ester
desNH$_2$  desamino
dl  diiodo
-NH2  amide
NPr  N-propyl
MeN  N-methyl
The first series of peptides of the present invention are the following:

3

### Peptide

VVNDL

NPrVVNDL

AVVNDL

AcAVVNDL

GAVVNDL

AcGAVVNDL

AGAVVNDL

YAGAVVNDL

AcYAGAVVNDL

YAGAVVNDL-NH$_2$

desNH2-YAGAVVNDL

AcYAGAVVNDL

dI-YAGAVVNDL

AcdI-YAGAVVNDL

SYAGAVVNDL

TSYAGAVVNDL

STSYAGAVVNDL

FPLSLMSTDKHTNFFECRSTSYAGAVVNDL

The second series of peptides of the present invention are the following:

4

```
              Y    V    V    N    D    U

              Y    V    V    N    D    L

            AcY    V    V    N    D    L

          MeO-Y    V    V    N    D    L

    desNH  -Y      V    V    N    D    L
         2
          MeN-Y    V    V    N    D    L

                        Bzl

              Y    V    V    N    D    L

              F    V    V    N    D    L

              H    V    V    N    D    L

              W    V    V    N    D    L

              Y    V    V    N    D    F

              Y    V    V    Q    D    L

              A    V    V    D    D    L

         Y    A    V    V    N    D    L

 desNH  -Y    A    V    V    N    D    L
      2
         Y    A    A    V    V    N    D    L

         Y    B    Y    V    V    N    D    L

         A    A    A    V    V    N    D    I

    F    A    G    A    V    V    N    D    L

    Y    A    G    A    V    V    J    D    L

    Y    A    P    A    V    V    N    D    L

    Y    A    G    Y    V    V    N    D    L    and

 K  K  L  E  E  F  L  K   V    V    N    D    L
```

The third series of peptides of the present invention are the following:

```
     A  V  V  N  dD  L

     A  V  V  dN  D  L

     A  V  dV  N  D  L

     dA  V  V  N  D  L

     dY  V  V  N  D  L

 Y A G A  V  V  dN  D  L

 Y A G dA  V  V  N  D  L

 Y A dA  A  V  V  N  D  L

 Y dA  G  A  V  V  N  D  L

 dY A  G  A  V  V  N  D  L
```

The polypeptides of the present invention and the amides and salts thereof can be manufactured according to known synthetic methods elongating the peptide chain, i.e. by condensing amino acids stepwise or coupling the fragments consisting of two to several amino acids, or by combination of both processes, or by solid phase synthesis according to the method originally described by Merrifield, J. Am. Chem. Soc. 85:2149-2154 (1963). Alternatively, the peptides of the present invention may be synthesized using automated peptide synthesized equipment.

The condensation between two amino acids, or an amino acid and a peptide, or a peptide and a peptide can be carried out according to the usual condensation methods such as azide method, mixed acid anhydride method, DCC (dicyclohexylcarbodiimide) method, active ester method (p-nitrophenyl ester method, N-hydroxysuccinic acid imido ester method, cyanomethyl ester method, etc), Woodward reagent K method, carbonyldiimidazol method, oxidation reduction method. These condensation reactions may be done in either liquid phase or solid phase. In the case of elongating the peptide chain in the solid phase method, the peptide is attached to an insoluble carrier at the C-terminal amino acid. For insoluble carriers, those which react with the carboxy group of the C-terminal amino acid to form a bond which is readily cleaved later, for example, halomethyl resin such as chloromethyl resin and bromomethyl resin, hydroxymethyl resin, aminomethyl resin, benzhydrylamine resin, and t-alkyloxycarbonylhydrazide resin can be used.

As is usual in peptide synthesis, it is necessary to protect/deprotect the $\alpha$- and $\omega$- side chain amino groups and the carboxy group of the amino acid as occasion demands. The applicable protective groups to amino groups are exemplified such as benzyloxycarbonyl (hereinafter abbreviated as Z), o-chlorobenzyloxycarbonyl [Z(2-Cl)], p-nitrobenzyloxycarbonyl [Z(NO2)], p-methoxybenzyloxycarbonyl [Z(OMe)], t-butoxycarbonyl (Boc), t-amyloxycarbonyl (Aoc), isobornyloxycarbonyl, adamantyloxycarbonyl, 2-(4-biphenyl)-2-propyloxycarbonyl (Bpoc), 9-fluorenylmethoxycarbonyl (Fmoc), methylsulfonylethoxycarbonyl (Msc), trifluoroacetyl, phthalyl, formyl, 2-nitrophenylsulphenyl (NPS), diphenylphosphinothioyl (Ppt), dimethylphosphinothioyl (Mpt) and the like.

As protective groups for carboxy group there can be exemplified, for example, benzyl ester (OBzl), 4-nitrobenzyl ester [OBzl(NO2)], t-butyl ester (OBut), 4-pyridylmethyl ester (OPic), and the like. It is desirable that specific amino acids such as arginine, cysteine, and serine possessing a functional group other than amino and carboxyl groups are protected by a suitable protective group as occasion demands. For example, the guanidino group in arginine may be protected with nitro, p-toluenesulfonyl, benzyloxycarbonyl, adamantyloxycarbonyl, p-methoxybenzenesulfonyl, 4-methoxy-2,6-dimethylbenzenesulfonyl (Mds), 1,3,5-trimethylphenylsulfonyl (Mts), and the like. The thiol group in cysteine may be protected with benzyl, p-methoxybenzyl, triphenylmethyl, acetylaminomethyl, ethylcarbamoyl, 4-methylbenzyl, 2,4,6-trimethylbenzyl (Tmb) etc, and the hydroxyl group in serine can be protected with benzyl, t-butyl, acetyl, tetrahydropyranyl etc.

Conventional methods of peptide synthesis as described, for example, by Schroder et al., "The Peptides", Vol. I Academic Press, 1965, or Bodanszky et al., "Peptide Synthesis", Interscience Publishers, 1966 or McOmie (ed.), "Protective Groups in Organic Chemistry", Plenum Press, 1973, or "The Peptides: Analysis Synthesis, Biology", 2 Chapter 1, by Barany et al., Academic Press, 1980, the disclosures of which are hereby incorporated by reference.

## EXAMPLE

### Preparation of Herpes Simplex Virus Ribonucleotide Reductase

HSV RR was purified from Baby Hamster Kidney cells that were cultured with HSV2 Strain 186. After infection, cells were harvested and lysed. Crude HSV RR was prepared as described by Huszar et al., J. Virol. 37:580 588 (1981), then subjected to further purification by hydroxyapatite column chromotography. The resultant enzyme preparation was about 80% pure HSV RR as judged by SDS polyacrylamide electrophoresis.

### Assay of Inhibition of HSV RR Activity

HSV RR activity was determined as the ability of the enzyme to catalyze the reduction of tritiated cytidine diphosphate to deoxyribo-cytidine-diphosphate in 30 minutes at 37°C in the presence of dithiothreitol and MgCl2. Activity of oligopeptide inhibitors was determined by adding them to the reaction mix at the beginning of the incubation. Enzyme was not preincubated with inhibitor.

### Synthesis, Purification, and Analysis of Oligopeptides

Oligopeptides were synthesized by either the solid phase method of Merrifield, op cit., or by FMOC chemistry (John Morrow Stewart and Janis Dillaha Young, "Solid Phase Peptide Synthesis," 2nd ed., 1984, Pierce Chemical Co., Rockford, Ill.) and purified to >98% homogeneity by reverse phase HPLC and recovered by lyophilization as trifluoroacetic acid salts. These were analyzed for amino acid composition and in some cases sequenced. Dry peptides were resuspended in 100 mM HEPES (pH 8.0) at about 2 mM, relyophilized, and stored in aliquots at -80°C. Immediately prior to use they were rehydrated with distilled $H_2O$ and centrifuged to remove any undissolved peptide. The concentrations of these stock solutions were then determined by measuring A291 nm at basic pH ($\varepsilon = 2.55$) for tyrosine-containing peptides and by quantitative amino acid composition for those peptides without tyrosine.

The first series of peptides were tested for ability to inhibit herpesvirus ribonucleotide reductase according to the foregoing method and the extent of the inhibition (μM concentration of peptide producing 50% inhibition of enzyme activity) is shown in the following table.

| Peptide | $IC_{50}$ ($\underline{HM}$) |
|---------|------|
| VVNDL | 250 |
| NPrVVNDL | 120 |
| AVVNDL | 51 |
| AcAVVNDL | 87 |
| GAVVNDL | 70 |
| AcGAVVNDL | 75 |
| AGAVVNDL | 80 |
| YAGAVVNDL | 10 |
| AcYAGAVVNDL | 5 |
| YAGAVVNDL–NH$_2$ | 75 |
| desNH2–YAGAVVNDL | 20 |
| AcYAGAVVNDL | 5 |
| dI–YAGAVVNDL | 20 |
| AcdI–YAGAVVNDL | 16 |
| SYAGAVVNDL | 17 |
| TSYAGAVVNDL | 17 |
| STSYAGAVVNDL | 17 |
| FPLSLMSTDKHTNFFECRSTSYAGAVVNDL | 8 |

The second series of peptides were tested for ability to inhibit herpes virus ribonucleotide reductase according to the foregoing method and the extent of the inhibition (μM concentration of peptide producing 50% inhibition of enzyme activity) is shown in the following table.

7

| | | | | | | | Peptide | | | | | | IC$_{50}$ (µM) |
|---|---|---|---|---|---|---|---|---|---|---|---|---|---|
| | | | | | | | Y | V | V N | D | U | | 55 |
| | | | | | | | Y | V | V | N | D | L | 15 |
| | | | | | | | AcY | V | V | N | D | L | 18 |
| | | | | | | | MeO–Y | V | V | N | D | L | 20 |
| | | | | | | | desNH$_2$–Y | V | V | N | D | L | 24 |
| | | | | | | | MeN–Y | V | V | N | D | L | 12 |
| | | | | | | | Y | V | V | N(Bzl) | D | L | 4.4 |
| | | | | | | | F | V | V | N | D | L | 65 |
| | | | | | | | H | V | V | N | D | L | 46 |
| | | | | | | | W | V | V | N | D | L | 17 |
| | | | | | | | A | V | V | D | D | L | 195 |
| | | | | | | desNH$_2$–Y | A | V | V | N | D | L | 160 |
| | | | | | A | A | A | V | V | N | D | L | 77 |
| | | | | | Y | A | A | V | V | N | D | L | 112 |
| | | | | | Y | B | Y | V | V | N | D | L | 20 |
| | | | | F | A | G | A | V | V | N | D | L | 56 |
| | | | | Y | A | G | A | V | V | J | D | L | 200 |
| | | | | Y | A | P | A | V | V | N | D | L | 14 |
| | | | | Y | A | G | Y | V | V | N | D | L | 8 |
| K | K | L | E | E | F | L | K | V | V | N | D | L | 200 |

The third series of peptides were tested for ability to inhibit herpesvirus ribonucleotide reductase according to the foregoing method and the extent of the inhibition (µM concentration of peptide producing 50% inhibition of enzyme activity) is shown in the following table.

| Peptide | $IC_{50}(\mu M)$ |
|---|---|
| A V V N dD L | 520 |
| A V V dN D L | 460 |
| A V dV N D L | 600 |
| dA V V N D L | 90 |
| dY V V N D L | 30 |
| Y A G A V V dN D L | >620 |
| Y A G dA V V N D L | 72 |
| Y A dA A V V N D L | 88 |
| Y dA G A V V N D L | (210) |
| dY A G A V V N D L | 40 |

**Claims**

1. A peptide having the amino acid sequence:

VVNDL

NPrVVNDL

AVVNDL

AcAVVNDL

GAVVNDL

AcGAVVNDL

AGAVVNDL

YAGAVVNDL

AcYAGAVVNDL

YAGAVVNDL–NH$_2$

desNH2–YAGAVVNDL

AcYAGAVVNDL

dI–YAGAVVNDL

AcdI–YAGAVVNDL

SYAGAVVNDL

TSYAGAVVNDL

STSYAGAVVNDL or

FPLSLMSTDKHTNFFECRSTSYAGAVVNDL

and the amides and physiologically acceptable salts thereof.

2. A composition comprising a peptide of Claim 1 in a pharmaceutically acceptable carrier.

3. A method of inhibiting a ribonucleotide reductase enzyme of herpes simplex virus <u>in vitro</u> comprising contacting the enzyme with a peptide of claim 1.

4. A peptide having the amino acid sequence:

```
                        Y   V   V   D   U,
                    Y   V   V   N   D   L,
                  AcY   V   V   N   D   L,
                MeO-Y   V   V   N   D   L,
           desNH -Y     V   V   N   D   L,
                2
                MeN-Y   V   V   N   D   L,


                                Bzl
                                 |
                    Y   V   V   N   D   L,
                    F   V   V   N   D   L,
                    H   V   V   N   D   L,
                    W   V   V   N   D   L,
                    Y   V   V   N   D   F,
                    Y   V   V   Q   D   L,
                    A   V   V   D   D   L,
                    A   V   V   J   D   L,
                Y   A   V   V   N   D   L,
       desNH -Y   A   V   V   N   D   L,
            2
            Y   A   A   V   V   N   D   L,
            Y   B   Y   V   V   N   D   L,
            A   A   A   V   V   N   D   I,
        F   A   G   A   V   V   N   D   L,
        Y   A   G   A   V   V   J   D   L,
        Y   A   P   A   V   V   N   D   L,
        Y   A   G   Y   V   V   N   D   L,  or
K   K   L   E   E   F   L   K   V   V   N   D   L
```

and the amides and physiologically acceptable salts thereof.

5. A composition comprising of a peptide of claim 4 in a pharmaceutically acceptable carrier.

6. A method of inhibiting a ribonucleotide reductase enzyme of herpes simplex virus <u>in vitro</u> comprising contacting the enzyme with a peptide of claim 4.

7. A peptide having the amino acid sequence:

```
       A  V  V  N  dD  L
       A  V  V  dN  D  L
       A  V  dV  N  D  L
      dA  V  V  N  D  L
      dY  V  V  N  D  L
   Y  A  G  A  V  V  dN  D  L
   Y  A  G  dA  V  V  N  D  L
   Y  A  dA  A  V  V  N  D  L
   Y  dA  G   A  V  V  N  D  L  or
  dY  A  G  A   V  V  N  D  L
```

and the amides and physiologically acceptable salts thereof.

8. A composition containing a peptide of Claim 7 in a pharmaceutically acceptable carrier.

9. A method of inhibiting a ribonucleotide reductase enzyme of herpes simplex virus <u>in vitro</u> comprising contacting the enzyme with a peptide of claim 7.